# EUROPEAN PATENT APPLICATION

(11) **EP 0 684 029 A2**
(43) Date of publication of application: **29.11.1995**
(21) Application number: 95302607.7
(22) Date of filing: 19.04.1995
(51) Int. Cl.: A61F 13/15

(54) **Body contoured disposable diaper having a sealable pocket**

(30) Priority: 26.04.1994 US 233275
(71) Applicant: Riceman, Robert G., West Caldwell, New Jersey 07006 (US); Levine, Andrea, West Caldwell, New Jersey 07006 (US); Medina, Mitchell A., Essex Fells, New Jersey 07021 (US)
(72) Inventor: Riceman, Robert G., West Caldwell, New Jersey 07006 (US); Levine, Andrea, West Caldwell, New Jersey 07006 (US); Medina, Mitchell A., Essex Fells, New Jersey 07021 (US)
(74) Representative: Perkins, Sarah

(57) **Abstract**

A body contoured disposable diaper having a sealable pocket. The diaper member has opposite end edges and opposite side edges, the side edges having leg accommodating recesses midway along the length thereof extending toward each other, the member being constituted by a moisture permeable plastic inner sheet, and a moisture impermeable plastic outer sheet sealed to the inner sheet around the edges thereof, and a moisture adsorbent material between the sheets. A pocket forming sheet is provided on the outside of the outer sheet extending from a position between the middle portion of one pair of the opposite edges to one edge of the other pair of opposite edges and having opposed pocket opening forming edges extending from one edge of one of the one pair of opposite edges to the opposite edge of the one pair of opposite edges across the entire transverse dimension of the diaper member and defining a pocket opening therebetween, the pocket opening forming edges having pocket sealing members extending therealong for, when the diaper member is folded over soil on the inner sheet and the pocket opening is opened at the pocket opening forming edges and the pocket forming sheet is drawn around the remainder of the diaper to be turned inside out and enclose the remainder of the diaper within a pocket, the sealing means can be sealed to seal the soiled diaper within the pocket.

## Description

### BACKGROUND OF THE INVENTION

The present invention relates to a body contoured disposable diaper which has a sealable pocket incorporated in the diaper structure and into which the diaper can be sealed after being folded over around soil on the inner face of the diaper.

Disposable diapers have been available for many years, and they generally include an inner sheet of moisture permeable plastic to be placed against the skin of a baby to be diapered, a moisture absorbent material filler against the outside of the inner sheet, and an outer moisture impermeable plastic sheet over the outside of the filler. The diaper is conventionally contoured by the provision of leg accommodating recesses along the side edges so as to fit around the legs of the baby, with the narrower portion of the diaper between the leg accommodating recesses extending through the crotch of the baby. At one of the opposite ends, there is usually provided some sort of fastening means, such as laterally outwardly extending adhesive tabs on the one opposite end which, when the diaper is placed in position around the lower body portion of the baby, are folded over and adhered to the other opposite end of the diaper.

The conventional way of disposing of such diapers which have been soiled by the baby is to simply fold the diaper over the soil, with the outer moisture impermeable plastic sheet on the outside, and then seal the adhesive tabs on the one end against the outer face of the thus folded over diaper.

While the thus folded up soiled diaper does not have any soil directly exposed to the exterior, the soil is not sealed inside the diaper, and leakage of the soil can occur, and, more frequently, the odor from the soil can escape. This creates unpleasant aroma conditions in the vicinity of the container into which the thus folded over diapers are temporarily stored before ultimate disposal thereof.

### OBJECT AND BRIEF SUMMARY OF THE INVENTION

It is the object of the present invention to provide a disposable diaper in which there is provided a pocket which can be formed by folding a part of the diaper around the soiled diaper and in which pocket the diaper can be sealed, so that soil cannot escape, and, likewise, the odor from the soil cannot escape.

It is a further object of the invention to provide such a diaper which contains only a single additional sheet of material, and which can, therefore, be easily manufactured on existing machinery for the manufacture of the conventional disposable diapers.

It is a further object of the invention to provide such a diaper which contains only a few additional sheets of material to form the pocket or a bag, and which can be easily manufactured on existing machinery.

It is a still further object of the invention to provide such a diaper in which the pocket is formed by a separate bag.

To this end, the disposable diaper of the present invention is constituted by a diaper member having opposite end edges and opposite side edges, the side edges having leg accommodating recesses approximately midway along the length thereof extending toward each other, and the diaper member being constituted by a moisture permeable plastic inner sheet, a moisture impermeable plastic outer sheet joined to the inner sheet around the edges thereof, and a moisture absorbent material between the sheets. At least one pocket forming sheet is provided on the outside of the outer sheet extending from a position between the middle portion of one pair of the opposite side edges to adjacent one edge of the other pair of opposite edges. This pocket forming sheet has an exposed pocket opening formed by pocket opening forming edges extending across the pocket forming sheet in a direction from one edge of the one pair of opposite edges toward the opposite edge of the one pair. The pocket opening forming edges have pocket sealing means extending therealong. When the diaper member is folded over soil on the inner sheet, the pocket is open at the pocket opening forming edges and the pocket forming sheet is drawn around the remainder of the diaper so that the pocket forming sheet is turned inside out to enclose the remainder of the diaper within a pocket formed by the inside out pocket forming sheet, and the sealing means is then sealed to seal the soiled diaper within the pocket.

As a result, the soiled diaper is sealed in the pocket so as to prevent escape of both the soil and the odor from the soil.

The pocket can be formed by a plurality of sheets, or can be formed by a separate bag.

### BRIEF DESCRIPTION OF THE DRAWINGS

The invention will now be described in greater detail in connection with the accompanying drawings, in which:
Fig. 1 is a plan view of a first embodiment of a body contoured disposable diaper having a sealable pocket;
Fig. 2 is a sectional view thereof taken along line 2-2 in Fig. 1;
Fig. 3 is a sectional view of the diaper similar to Fig. 2, but showing the diaper folded over to contain soil therewithin;
Fig. 4 is a view similar to Fig. 3 showing the diaper after the pocket forming layer has been drawn around the remainder of the diaper so as to be turned inside out and the edges sealed to seal the soil within the pocket formed by the inside out sheet;
Fig. 5 is a plan view of a second embodiment of a body contoured disposable diaper having a sealable pocket according to the present invention;
Fig. 6 is a sectional view taken along line 6-6 of Fig. 5;
Fig. 7 is a sectional view of the diaper after it has been folded and the pocket forming sheet turned inside out to enclose the folded diaper within the pocket;
Fig. 8 is a schematic view of an apparatus for producing a diaper according to the present invention;
Fig. 9 is a plan view of a third embodiment of a disposable diaper having a sealable pocket according to the invention;
Fig. 10 is a sectional view taken on line 10-10 of Fig. 9;
Fig. 11 is a plan view of a further embodiment of a disposable diaper having a sealable pocket according to the invention;
Fig. 11a is a plan view of a further embodiment of a disposable diaper having a sealable pocket according to the invention which is a modified form of the embodiment of Fig. 11;
Fig. 11b is a plan view of a further embodiment of a disposable diaper having a sealable pocket according to the invention and which is a further modified form of the embodiment of Fig. 11;
Fig. 12 is a plan view of another embodiment of a disposable diaper having a sealable pocket according to the invention;
Fig. 13 is a sectional view taken along line 13-13 of Fig. 12;
Fig. 14 is a plan view of still another embodiment of a disposable diaper having a sealable disposal container constituting the product according to the invention;
Fig. 15 is a sectional view taken along line 15-15 of Fig. 14;
Fig. 16 is a sectional view of the container of Figs. 14 and 15 with a soiled diaper therein and the opening closed by a closure flap; and
Fig. 17 is a view similar to Fig. 16 showing a modified embodiment similar to that of Fig. 14.

### DETAILED DESCRIPTION OF THE INVENTION

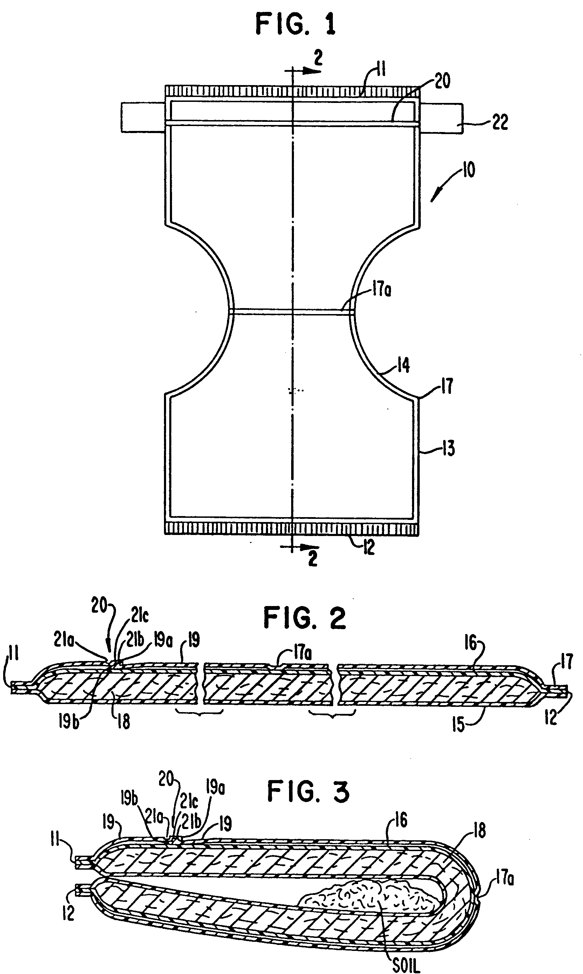

As shown in Figs. 1-4, the first embodiment of the body contoured disposable diaper having a sealable pocket is constituted by a diaper member generally indicated at 10 having opposite side edges 13 with leg accommodating recesses 14 therein approximately midway along the longitudinal dimension thereof, with the recesses extending toward each other to define a narrow portion at the center of the length of the diaper which is positioned in the crotch of the baby when the diaper is applied to the baby. The diaper member 10 has a moisture permeable plastic inner sheet 15 and a moisture impermeable plastic outer sheet 16 sealed to the inner sheet around the edges thereof at 17, by a heat seal, for example. A moisture absorbent material filler 18 is provided between the inner sheet and the outer sheet. At one end, near one end edge 11, there is provided a plurality of laterally outwardly extending adhesive tabs 22, or other fastening means which, when the diaper is applied to the baby, are folded over the outer face of the opposite end of the diaper to hold the ends of the diaper around the waist of the baby being diapered.

The diaper member thus far described is typical of conventional disposable diapers presently available.

The present invention incorporates in this diaper member structure a pocket forming sheet 19 preferably of moisture impermeable material, such as a moisture impermeable plastic, which is placed against the outside of the outer sheet 16, and it extends at least from a position between the middle portion of one pair of opposite edges, in this case the opposite side edges 13, to adjacent one edge of the other pair of opposite edges, in this case one of the end edges 11. It is sealed to the outer sheet 16 along a seam 17a, for example a heat seal, extending between recesses 14 and along the seal 17 around the edges of sheets 15 and 16.

The pocket forming sheet 19 has opposed pocket opening forming edges 19a and 19b extending from one edge of one of the one pair of opposite edges, namely from one side edge 13, across the entire transverse dimension of the pocket forming sheet 19. The pocket opening forming edges define between them a pocket opening 20. Extending along the pocket opening forming edges 19a and 19b are pocket sealing means, here shown as a double sided groove and rib type seal with a groove 21a on the outer face of the pocket forming sheet 19 in which is engaged a rib 21b on the outer face of the pocket forming sheet 19. There may also be a second groove 21c in the inner face of the part with groove 21a thereon. These elements, the grooves 21a and 21c and the rib 21b when they are juxtaposed with the rib 21b extending into the groove 21a or groove 21c in a sealing frictional fit form a seal therebetween. In the unused diaper, rib 21b may be engaged in groove 21c to seal the pocket opening when groove 21c is present. In actual practice the pocket forming sheet 19 may also extend from the seam 17a to the other end edge 12, and be sealed to the sheet 16 along the remainder of the seal 17. This is due to the manufacturing process. The essential part of the pocket forming sheet 19 is that which extends from seam 17a to end edge 11.

In use, after the diaper with soil on the inner sheet 15 is removed from the baby, it is first folded over so that the one end edge 11 is against the other end edge 12, as shown in Fig. 3. In the conventional manner of disposing of such a diaper, with the diaper in this condition, the adhesive tabs 22 which have previously been adhered to the outer sheet to hold the diaper on the baby and then peeled off the outer sheet to enable removal of the diaper from the baby, are folded over the outer face of the outer sheet and readhered thereto to hold the diaper in the folded condition.

The manner of preparing the diaper of the present invention for disposal involves the folding of the soiled diaper in the folded condition of Fig. 3 into a pocket formed by the pocket forming sheet 19.

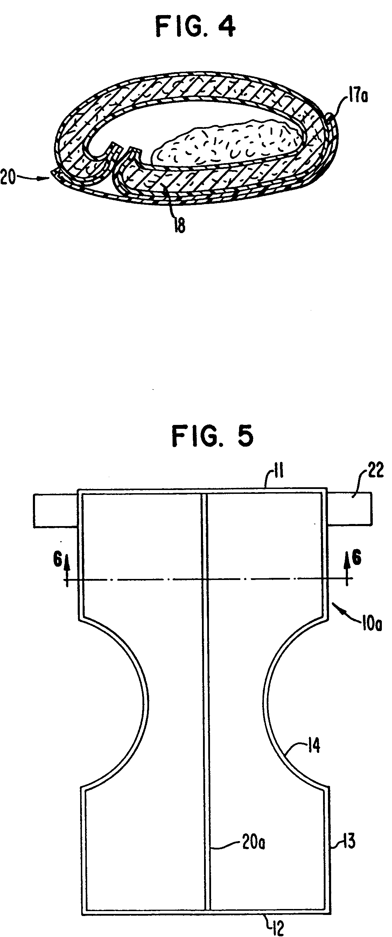

To accomplish this, the opposed pocket opening forming edges 19a and 19b are separated and the edges are gripped at the pocket opening 20, and the pocket forming sheet is drawn around the remainder of the diaper to, in effect, turn the pocket forming sheet inside out so that the pocket forming sheet extends around the entire outside of the folded over diaper member across the gap between the opposite end edges 11 and 12 of the folded diaper, to the position as shown in Fig. 4, in which the sealing means along the opposed pocket forming edges 19a and 19b are engageable with each other. In the present embodiment, the rib 21b will be in spaced opposed relationship to the groove 21a, and can be readily inserted into groove 21a so as to form a friction fit and thus seal the opposed pocket forming edges to each other. As a result, the diaper will be folded into a sealed package in which the outer surfaces are constituted on the one side of the folded diaper by the outer moisture impermeable plastic sheet 16, and on the other side by the pocket forming sheet 19.

It will be understood that because the edges of the pocket opening are sealed to each other, not only can the soil be prevented from escaping, but the odor will also be prevented from escaping.

Alternatively, the pocket forming sheet 19 can extend less than the entire transverse width of the diaper member 10, and the edges can be sealed to the outer sheet 16 instead of in the seal 17.

The second embodiment as shown in Figs. 5-7 is essentially the same as the first embodiment, and the parts which are the same as in the first embodiment are identified by the same reference numerals.

The diaper member 10a has the inner sheet of moisture permeable plastic 15, the absorbent material filler 18 and the outer moisture impermeable plastic sheet 16 sealed to the edges of the inner sheet, just as in the diaper member of Fig. 1.

The pocket forming sheet 19a, however, instead of having the pocket opening extending transversely to the diaper from one side edge 13 to the other, has the pocket forming opening 20a extending from one end edge 11 to the other end edge 12. A rib and groove pocket sealing means 21a, 21b is provided along this opening 20a.

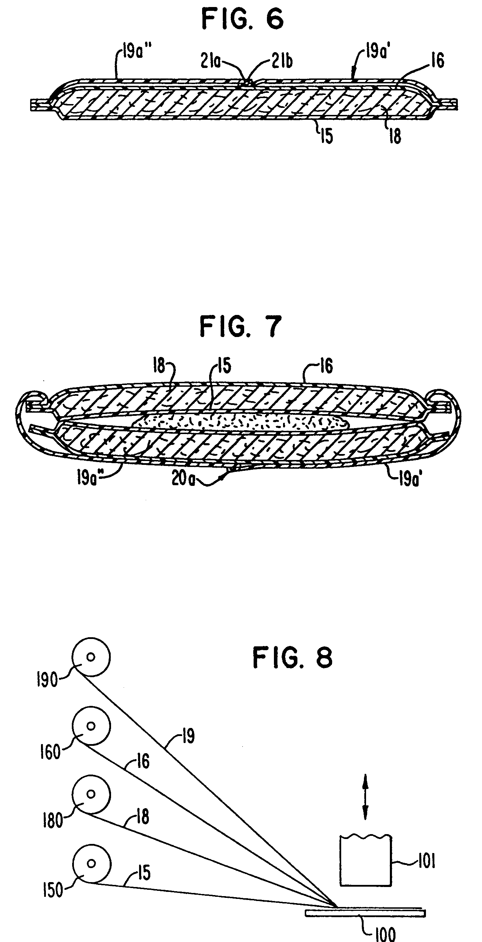

After the diaper has been folded in the same manner as shown in Fig. 3, i.e. with the opposite end edges 11 and 12 against each other, with the soil contained within the folded diaper, the opposite halves 19a' and 19a'' of the pocket forming sheet 19a are pulled outwardly and around the outside of the thus folded over diaper member to form a pocket and the opposite edges are sealed as shown in Fig. 7, in the same manner as in Fig. 4. As shown in Fig. 7, the folded soiled diaper is thus enclosed within the moisture impermeable plastic layer 16 and the inside out pocket forming sheet constituted by the halves 19a' and 19a''. Again, as with the embodiment of Figs. 1-4, the soil is sealed within the enclosure, and the odor is likewise sealed within the enclosure.

It will be seen that in this embodiment only a single side rib and groove sealing means has been used, not a double sided one as shown in the embodiment of Figs. 1-4.

Further, the pocket forming sheet 19a and the opening 20a can extend less than the entire length of the diaper 10a.

The diaper according to the present invention can be easily manufactured on conventional apparatus for forming diapers of this type.

The basic diaper member itself is formed by leading a sheet 15 of moisture permeable plastic from a supply roll 150 thereof across a platen 100, overlying such sheet by a sheet 18 of absorbent material filler 18 from a supply roll 180 thereof, and then overlying the sheets 15 and 18 with a sheet 16 of moisture impermeable plastic from a supply roll 160, and then stamping the stack of sheets with a heated die 101 against a platen 100 to cut out the diaper and seal the edges of the sheets to each other.

To manufacture the diaper according to the present invention, it is only necessary to provide a supply roll 190 of successive pocket forming sheets 19 previously formed so as to have the necessary pocket forming opening therein at the desired position and with the sealing means along the edges thereof. This sheet is overlaid on the stack of the inner sheet, the absorbent material filler and the outer sheet, and is stamped and heat sealed to this stack of sheets at the same time as the stack of sheets themselves are cut and sealed, thus forming the diaper according to the present invention.

In the first two embodiments, the pocket into which the soiled diaper is enclosed is formed from the moisture impermeable plastic outer sheet 16 and the single pocket forming sheet 19. It is possible to provide a pocket which does not use the plastic outer sheet of the diaper as an outer sheet.

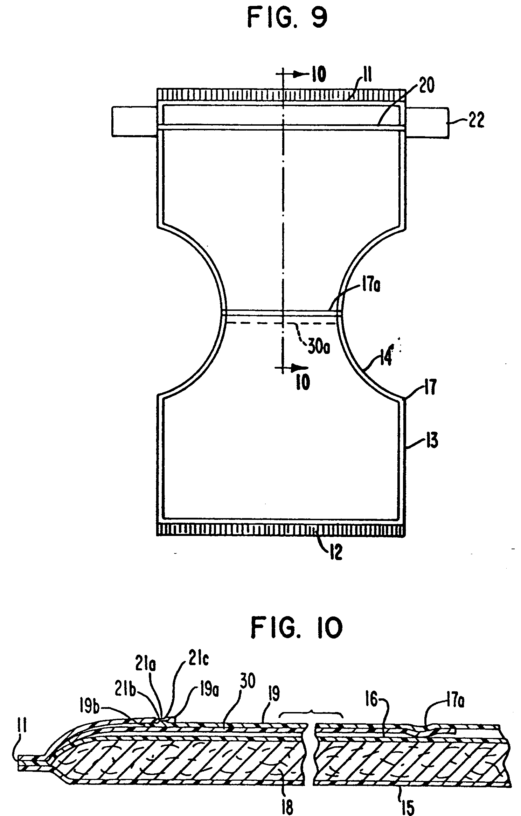

In the third embodiment, as shown in Figs. 9 and 10, the structure is the same as the embodiment of Figs. 1-4, but there has been added an inner pocket forming sheet 30 of moisture impermeable material, such as moisture impermeable plastic. This sheet is secured in the diaper along the edges at 17 and preferably ends just below the seam 17a, as shown by the dotted line 30a, although it can extend to the end 12 just like the pocket forming sheet 19. It defines a space between itself and the pocket forming sheet 19 to which access is gained through the opening 20, and in the normal use of the diaper is closed by sealing means, again shown in the form of the double sided groove and rib seal 21a, 21b, 21c.

This embodiment is used in the same way as the embodiment of Figs. 1-4. After the soiled diaper is removed from the baby and folded up, the seal means is opened to open the pocket opening 20 to give access to the pocket. The edges of the opening are grasped and the pocket is essentially turned inside out around the rolled up diaper and the seal means is sealed to close the opening 20. In this case, the rib 21b is frictionally inserted into the groove 21a which after the turning inside out of the pocket is on the outside of the pocket the same as is shown in Fig. 4. In the final condition of the diaper, the inner pocket forming sheet covers the outer sheet 16 where it is exposed as shown in Fig. 4.

The manufacture of this embodiment on the apparatus of Fig. 8 is similar to the manufacture of the first two embodiments. If the inner pocket forming sheet 30 is a full sheet, i.e. coextensive with sheet 19, it can be supplied from a separate roll positioned between the rolls 160 and 190, or can be rolled up on roll 190 together with the sheet 19. If it is to end just below the seam 17a, then successive ones must be temporarily tacked to the under side of the sheet 19 at positions where it will properly underlie the preformed openings 20 in sheet 19.

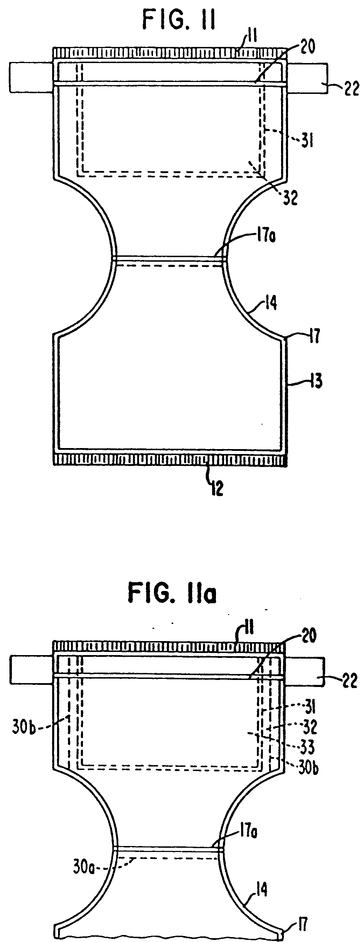

In the embodiment of Fig.11, the inner pocket forming sheet 30 is secured against the inside surface of the pocket forming sheet 19 by a junction along a line 31 which extends across the opposite end portions of the pocket opening 20 preferably to the seal 17 along the end edge 11. The junction is formed by a seal, such as formed by plastic welding or the like, to seal the sheets 19 and 30 along the line 31 to form a sealed space 32 constituting a pocket between the pocket forming sheet 19 and the inner pocket forming sheet 30. The pocket opening 20 in the pocket forming sheet 19 gives access to the space 32 and in the condition of normal use of the diaper is closed by sealing means, again shown in the form of the double sided groove and rib seal 21a, 21b, 21c. The end of the inner pocket forming sheet remote from the end edge 11 is preferably sealed between the pocket forming sheet 19 and the plastic outer sheet 16 at the seam 17a.

The manufacture of this embodiment on the apparatus of Fig. 8 is similar to the manufacture of the first and second embodiments. However, on roll 190, instead of the simple pocket forming sheet, there is wound a pocket forming sheet to the lower side of which has been attached the inner pocket forming sheets by the welding along line 31 and at intervals such that as the sheet 19 is brought against the remainder of the parts of the diaper, the formed spaces 32 will be under the openings 20 and in position adjacent the upper edges 11 of the successively formed diapers.

The advantage of the provision of the additional pocket forming sheet in this embodiment is that it can be made of stronger or more water impervious material than the outer sheet 16 of the diaper.

The inner pocket forming sheet 30 need not extend to the edges of the diaper. It can end inwardly thereof, as shown by the dotted lines 30b in Fig. 11a. In such an embodiment, the sheet 30 must be secured to the remainder of the diaper structure by means other than the edge seals 17. In this embodiment, the sheet is secured against the inside surface of the pocket forming sheet 19 by a junction along a line 31 which extends across the opposite end portions of the pocket opening 20 preferably to the seal 17 along the end edge 11. The junction is formed by, for example, plastic welding or the like, to seal the sheets 19 and 30 along the line 31 to form a sealed space 32 between the pocket forming sheet 19 and the inner pocket forming sheet 30. The pocket opening 20 in the pocket forming sheet 19 gives access to the space 32 and in the condition of normal use of the diaper may be closed by sealing means, again shown in the form of the double sided groove and rib seal 21a, 21b, 21c. The end of the inner pocket forming sheet remote from the end edge 11 is preferably sealed between the pocket forming sheet 19 and the plastic outer sheet 16 at the seam 17a.

The manufacture of this embodiment on the apparatus of Fig. 8 is similar to the manufacture of the first and second embodiments. However, on roll 190, instead of the simple pocket forming sheet, there is wound a pocket forming sheet to the lower side of which has been attached the inner pocket forming sheets by welding or other means along line 31 and at intervals such that as the sheet 19 is brought against the remainder of the parts of the diaper, the formed spaces 32 will be accessible through the openings 20 and in position adjacent the upper edges 11 of the successively formed diapers.

The advantage of the provision of the additional pocket forming sheet in this embodiment is that it can be made of stronger or more water impervious material than the outer sheet 16 of the diaper.

A still further embodiment is shown in Fig. 11b. This embodiment is like that of Fig.11 in that there is provided the separate space 32 between a pocket forming sheet and an inner pocket forming sheet. However, the plastic welding line 31 welding the inner pocket forming sheet to the pocket forming sheet is extended at 31a parallel to the end edge 11 in the area between the pocket opening 20 and the end edge, and around the outside of the weld line 31 is formed a score line extending through the pocket forming sheet 19 and the inner pocket forming sheet 30 so as to enable easy separation of the pocket, constituted by a bag enclosing the space 32 from the pocket forming sheets. As with the attachment of the inner pocket forming sheets 30 to the inside surface of the pocket forming sheet 19 prior to assembling all the sheets into the diaper, the score lines can also be formed prior to assembly. In this case, however, a double sided rib and groove sealing means is not needed. Rather, a conventional single rib on the outside of one edge of sheet 19 and a single groove on the inside of the other edge can be used.

In use, instead of the soiled diaper being sealed in the pocket formed by turning the pocket forming sheet 19 inside out around the rolled up soiled diaper, as in the use of the embodiment of Figs. 9 and 10, and Fig. 11a, the bag is first removed from the outside of the diaper by separating it from the pocket forming sheets along the score lines, and then the rolled up diaper is inserted into the separated bag and the bag sealed by the seal means along the opening 20.

The advantage of this embodiment is that the separate sealable bag is incorporated into the diaper as it is manufactured and accompanies the diaper during use so that it is always available to receive the soiled diaper when the time comes.

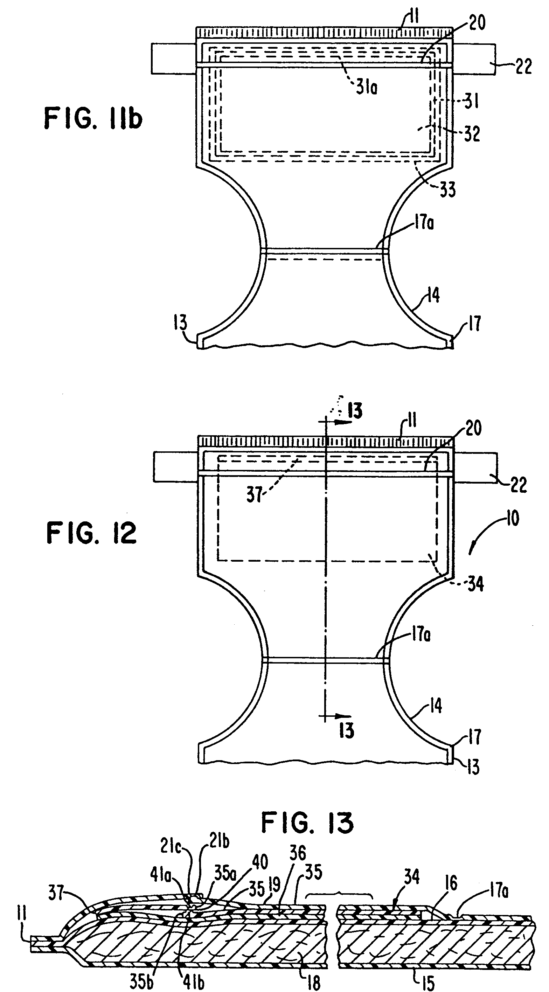

A separate pocket forming bag can be incorporated into the diaper structure as shown in the embodiment of Figs. 12 and 13. In this embodiment, a separately formed bag 34 in the form of a bag of moisture impermeable material, such as a bag of moisture impermeable plastic, is provided between the sheet 19, which no longer forms a part of the pocket and therefore need not be moisture impermeable, and the outer sheet 16. It is preferably attached to the inside surface of the sheet 19 along a weld line 37, and is formed of an outer bag sheet 35 and an inner bag sheet 36 which are integrally joined around the edges, or may be welded to each other around the edges as is conventional in forming plastic bags. It has sealing means along an opening 40 which is substantially in register with the opening 20 in the plastic sheet 19, and in the present embodiment the sealing means is a special rib and groove sealing means having a groove 41a on the outer side of one edge 35a of the outer bag sheet 35 and a rib 41b on the outer side of the other edge. As will be seen from the description of how this embodiment is used, the groove 41a and the rib 41b must both be on the outside of the sheet 35 since the bag is essentially turned inside out in use to form the. diaper-receiving pocket, and the groove must be available on the outside of the sheet to receive the rib. Since this sealing means is what seals the pocket with the soiled diaper therein, a simple single rib and groove sealing means 21b,21c can be used for closing the opening 20 in the outer pocket forming sheet 19.

In use, the soiled diaper is folded up, and then the opening 20 is opened by separating the edges 19a and 19b to separate them, and the bag 34 is opened by separating the edges 35a and 35b to separate the sealing means. These edges are then moved around the soiled diaper to turn the bag 34 inside out to form the diaper-receiving pocket in the same manner as in the embodiment of Figs. 1-4, and the sealing means is sealed by inserting the rib 41b into the groove 41a.

To manufacture this embodiment in the apparatus of Fig. 8, the preformed bags 34 are attached at intervals along the sheet 19 with the opening in the bag just within the opening 20 in the sheet 19. As described above, the attachment can be by means of a weld 37 along the top edge of the bag 34 which lies between the point where the edge 11 will be formed and the opening 20.

The advantage of this embodiment is that preformed bags are used which are attached to the diaper structure by a single simple weld.

This embodiment can be modified by securing the bag 34 by a separable securing means, for example by making the weld 37 a separable weld, which enables the bag 34 to be separated from the inside surface of the sheet 19 by simply pulling the bag and the sheet apart. In this case, the bag 34 then becomes a separate bag, like the bag 32 of the embodiment of Fig. 11 into which the soiled diaper is placed, and then the edges of the opening in the bag are sealed by a sealing means. Where the sealing means is the rib and groove sealing means, a conventional single rib and groove like that shown for the sheet 19 in Fig. 13 will suffice.

The description of the rib and groove sealing means has recited grooves on respective outer and inner surfaces and ribs on particular outer surfaces. It will be appreciated that the ribs can be replaced by grooves and vice versa in all instances without exceeding the scope of the invention. For example, in Fig. 2, the grooves 21a and 21c can be replaced by ribs, and the rib 21b can be replaced by a groove, and the sealing means will operate in exactly the same way as the specific form as shown.

Sealing means other than the rib and groove sealing means can be used, for example, adhesive tape type sealing means.

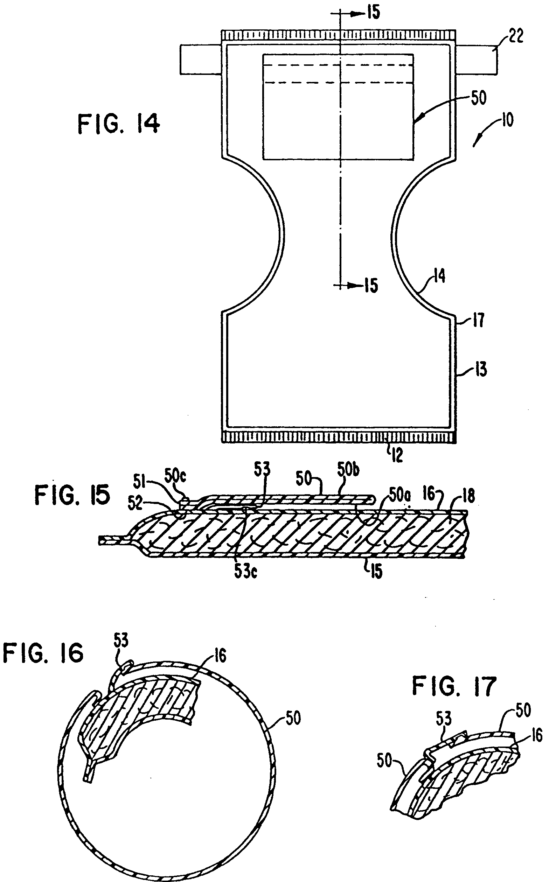

A separate pocket forming bag can be mounted on the outside of the diaper structure as shown in the embodiment of Figs. 14 and 15. In this embodiment, a separately formed bag 50 in the form of a sheet of moisture impermeable material formed into a bag having an inner and outer bag forming sheet 50a and 50b having edges defining a bag opening 51, such as a bag of moisture impermeable plastic, is secured along a part of the inner bag forming sheet adjacent the upper end at 52 to the outer sheet 16 of the diaper with an upper opening 51 of the bag exposed. Along an outer bag forming sheet 50b on the other face of the bag adjacent the upper opening 51 is one part of a sealing means, which in the embodiment shown is a rib 50c forming part of a rib and groove sealing means such as described hereinabove. Beneath the upper end of the bag 50 is provided a closure flap 53 having the edge corresponding to the upper end of the bag 50 sealed to the outer sheet 16 of the diaper, and extending away from the bag opening and ending in a free edge 53a having thereon the other part of the sealing means, here shown as a groove 53c of the rib and groove sealing means, on the surface thereof facing the outer sheet 16.

In use, the soiled diaper is folded up and then the folded up diaper is folded around the top edge of the inner bag forming sheet 50a to be inserted into the bag. Then the closure flap 53 is placed across the opening of the bag and the sealing means 53c on the closure flap 53 is engaged with the sealing means 50c on outer bag forming sheet 53b to close and seal the bag, as shown in Fig. 16.

Alternatively, the closure flap 53 can be secured to the inner bag forming sheet 50a as shown in Fig. 17, and after the soiled diaper is inserted into the bag, it is folded over the top of the bag to engage the sealing means 50c, 53c.

It will thus be seen that there has been provided a body contoured disposable diaper having a pocket formed thereon into which the folded soiled diaper is folded so as to seal the soiled diaper within the pocket, effectively sealing the diaper package against escape of soil as well as the odor from the soil. The diaper according to the invention can be manufactured on conventional apparatus, so that it is easy and inexpensive to manufacture.

## Claims

1. A disposable diaper having a sealable pocket, comprising:
a diaper member having opposite end edges and opposite side edges, said diaper member being constituted by a moisture permeable inner sheet, and a moisture impermeable outer sheet joined to said inner sheet around the edges thereof, a moisture absorbent material between said sheets; and
at least one pocket forming sheet of moisture impermeable material on the outside of said outer sheet extending at least from a position between the middle portion of one pair of said opposite edges to adjacent one edge of the other pair of opposite edges and having opposed pocket opening forming edges extending across said pocket forming sheet in a direction from one edge of said one pair of opposite edges toward the opposite edge of said one pair of opposite edges and defining a pocket opening therebetween, said pocket opening forming edges having pocket sealing means extending therealong for, when the diaper member is folded over soil on the inner sheet and the pocket opening is opened at said pocket opening forming edges and said pocket forming sheet is drawn around the remainder of said diaper to be turned inside out and enclose the remainder of the diaper within a pocket, said sealing means can be sealed to seal the soiled diaper within the pocket.

2. A diaper as claimed in claim 1 in which said pocket forming sheet extends from a line between a position approximately midway along said side edges to adjacent one end edge, and said pocket opening forming edges extend between opposite side edges.

3. A diaper as claimed in claim 2 in which said pocket forming sheet is sealed to said outer sheet along said line between said positions approximately midway along said side edges to form a bottom of said pocket.

4. A diaper as claimed in claim 1 in which said pocket forming sheet extends between said side edges, and said pocket forming edges extend between said opposite end edges.

5. A diaper as claimed in claim 1 further comprising an inner pocket forming sheet of a moisture impermeable material between said outer sheet and said pocket forming sheet, said inner pocket forming sheet being joined to the diaper to enclose a space between said inner pocket forming sheet and said pocket forming sheet, whereby when said pocket forming sheet is drawn around the soiled diaper said pocket forming sheet and said inner pocket forming sheet together enclose the soiled diaper in the pocket.

6. A diaper as claimed in claim 5 in which said inner pocket forming sheet is coextensive with said pocket forming sheet within an area in which said pocket forming sheet and said inner pocket sheet are joined to said diaper.

7. A diaper as claimed in claim 5 in which said inner pocket forming sheet is joined to said pocket forming sheet along a line defining a pocket forming space between said pocket forming sheet and said inner pocket forming sheet to which access can be gained through said opposed pocket forming edges in said pocket forming sheet.

8. A diaper as claimed in claim 5 in which said inner pocket forming sheet is less than coextensive with said pocket forming sheet within an area in which said pocket forming sheet and said inner sheet are joined to said diaper, and said inner pocket forming sheet is joined to said pocket forming sheet along a line defining a pocket forming space between said pocket forming sheet and said inner pocket forming sheet to which access can be gained through said opposed pocket forming edges in said pocket forming sheet.

9. A disposable diaper having a sealable bag incorporated therein, comprising:
a diaper member having opposite end edges and opposite side edges, said diaper member being constituted by a moisture permeable inner sheet, a moisture impermeable outer sheet joined to said inner sheet around the edges thereof, and a moisture absorbent material between said sheets;
a pocket forming sheet of moisture impermeable material on the outside of said outer sheet extending at least from a position between the middle portion of one pair of said opposite edges to adjacent one edge of the other pair of opposite edges and having opposed pocket opening forming edges extending across said pocket forming sheet in a direction from one edge of said one pair of opposite edges to the opposite edge of said one pair of opposite edges and defining a pocket opening therebetween, said pocket opening forming edges having pocket sealing means extending therealong; and
an inner pocket forming sheet of a moisture impermeable material between said outer sheet and said pocket forming sheet, said inner pocket sheet being joined to said pocket forming sheet along a line defining a pocket forming space between said pocket forming sheet and said inner pocket forming sheet to which access can be gained through said opposed pocket forming edges in said pocket forming sheet, said pocket forming sheet and said inner pocket forming sheet having a score line therein along an outer periphery of said line defining said space and along which score line said pocket forming sheet and said inner pocket forming sheet can be separated to separately remove parts of said pocket forming sheet and said inner pocket forming sheet within said line as a separate bag into which the diaper can be inserted when the diaper has become soiled and in which the diaper is sealed by sealing of said sealing means along said pocket opening forming edges.

10. A disposable diaper having a sealable bag incorporated therein, comprising:
a diaper member having opposite end edges and opposite side edges, said diaper member being constituted by a moisture permeable inner sheet, a moisture impermeable outer sheet joined to said inner sheet around the edges thereof, and a moisture absorbent material between said sheets;
a pocket forming sheet of moisture impermeable material on the outside of said outer sheet extending at least from a position between the middle portion of one pair of said opposite edges to adjacent one edge of the other pair of opposite edges and having opposed pocket opening forming edges extending across said pocket forming sheet in a direction from one edge of said one pair of opposite edges to the opposite edge of said one pair of opposite edges and defining a pocket opening therebetween, said pocket opening forming edges having pocket sealing means extending therealong; and
a pocket forming bag secured between said outer sheet and said pocket forming sheet, said bag having an inner and an outer bag forming sheet of moisture impermeable material joined to each other for forming a bag, and one of said bag sheets having a sealable opening therein through which access to the interior of said bag can be gained, said bag lying inside the pocket opening in said pocket forming sheet.

11. A diaper as claimed in claim 10 in which said bag is secured to said diaper by a separable securing means.

12. A diaper as claimed in claim 10 in which said bag is secured to the inside of said pocket forming sheet.

13. A diaper as claimed in claim 12 in which said bag is secured by a separable securing means.

14. A disposable diaper having a sealable bag combined therewith, comprising:
a diaper member having opposite end edges and opposite side edges, said diaper member being constituted by a moisture permeable inner sheet, a moisture impermeable outer sheet joined to said inner sheet around the edges thereof, a moisture absorbent material between said sheets;
a pocket forming bag having an inner and outer bag forming sheet of moisture impermeable material joined to each other for forming the bag, said bag having bag opening defined between said inner and outer sheets adjacent one end thereof, said bag being secured to said outer sheet; and
sealing means along said bag opening for sealing said bag opening after the soiled diaper has been folded over and inserted into said bag.

15. A disposable diaper having a sealable bag combined therewith, comprising:
a diaper member having opposite end edges and opposite side edges, said diaper member being constituted by a moisture permeable inner sheet, a moisture impermeable outer sheet joined to said inner sheet around the edges thereof, a moisture absorbent material between said sheets;
a pocket forming bag having an inner and outer bag forming sheet of moisture impermeable material joined to each other for forming the bag, said bag having a bag opening defined between said inner and outer sheets adjacent one end thereof, said bag being secured to said outer sheet;
a closure flap between said bag and said outer sheet and secured to one of said bag and said outer sheet and extending away from said bag opening and ending in a free edge; and
sealing means along the free edge of said closure flap and said outer bag forming sheet for sealing said bag opening after the soiled diaper has been folded over the edge of said inner bag sheet at said bag opening and inserted into said bag and said closure flap has been placed across the bag opening.
